# EUROPEAN PATENT SPECIFICATION

(11) **EP 3 035 942 B1**
(45) Date of publication and mention of the grant of the patent: **14.12.2022**
(21) Application number: 14838610.5
(22) Date of filing: 19.08.2014
(51) Int. Cl.: A61K 33/04, A61K 31/08, A61K 33/00, A61M 16/01, A61M 16/10, A61M 5/00, A61P 23/00, A61P 39/00

(54) **THE NON-ANESTHETIC PROTECTIVE GAS ARGON COMBINATION WITH LIQUID ANESTHETIC AGENTS FOR USE IN ORGAN PROTECTION**
DAS NICHT NARKOTISCHE SCHUTZGAS ARGON IN KOMBINATION MIT FLÜSSIGEN NARKOSEMITTELN ZUR MEDIZINISCHEN VERWENDUNG FÜR ORGANSCHUTZ
LE GAZ PROTECTEUR NON ANESTHÉSIQUE ARGON COMBINÉ À DES LIQUIDES ANESTHÉSIQUES, ET SON UTILISATION POUR LA PROTECTION DES ORGANES

(30) Priority: 19.08.2013 US 201361867367 P
(43) Date of publication of application: 29.06.2016
(73) Proprietor: Schmidt, Klaus Michael, Halifax, Nova Scotia B3H 4S9 (CA); Roach, David Cecil, Halifax, Nova Scotia B3H 3S3 (CA)
(72) Inventor: Schmidt, Klaus Michael, Halifax, Nova Scotia B3H 4S9 (CA); Roach, David Cecil, Halifax, Nova Scotia B3H 3S3 (CA)
(74) Representative: Dehns
(86) International application number: PCT/CA2014/000630
(87) International publication number: WO 2015/024100

(56) References cited:
- WO-A1-2011/098698
- WO-A1-2011/098698
- DE-A1- 10 161 252
- DE-A1- 19 833 014
- L E MCCRACKEN ET AL: "Ketamine and thiopental sleep responses in guinea pigs in hyperbaric helium-oxygen.", UNDERSEA BIOMEDICAL RESEARCH, vol. 6, no. 4, 1 December 1979 (1979-12-01), pages 329-339, XP055353014, US ISSN: 0093-5387
- K. KITAGUCHI ET AL: "Effects of sevoflurane on cerebral circulation and metabolism in patients with ischemic cerebrovascular disease.", ANESTHESIOLOGY., vol. 79, no. 4, 1 October 1993 (1993-10-01), pages 704-709, XP055353774, PHILADELPHIA, PA, US ISSN: 0003-3022
- F. STAFFIERI ET AL: "Pulmonary gas exchange in anaesthetised horses mechanically ventilated with oxygen or a helium/oxygen mixture", EQUINE VETERINARY JOURNAL., vol. 41, no. 8, 1 November 2009 (2009-11-01), pages 747-752, XP055353026, GB ISSN: 0425-1644, DOI: 10.2746/042516409X416198
- COBURN M ET AL: "The neuroprotective effects of xenon and helium in an in vitro model of traumatic brain injury", CRITICAL CARE MEDICINE, LIPPINCOTT WILLIAMS & WILKINS, US, vol. 36, no. 2, 1 January 2008 (2008-01-01), pages 588-595, XP008094471, ISSN: 0090-3493, DOI: 10.1097/01.CCM.0B013E3181611F8A6
- YI PAN ET AL: "The effect of heliox treatment in a rat model of focal transient cerebral ischemia", NEUROSCIENCE LETTERS, ELSEVIER, AMSTERDAM, NL, vol. 497, no. 2, 19 April 2011 (2011-04-19), pages 144-147, XP028218386, ISSN: 0304-3940, DOI: 10.1016/J.NEULET.2011.04.048 [retrieved on 2011-04-27]
- NADAUD J ET AL: "Association helium-sevoflurane : une therapeutique de sauvetage dans l'asthme aigu grave", ANNALES FRANCAISES D'ANESTHESIE ET DE REANIMATION, MASSON, PARIS, FR, vol. 28, no. 1, 1 January 2009 (2009-01-01), pages 82-85, XP025893723, ISSN: 0750-7658, DOI: 10.1016/J.ANNFAR.2008.10.022 [retrieved on 2009-01-13]
- LOETSCHER, PHILIP D. ET AL.: 'Argon: Neuroprotection in in vitro models of cerebral ischemia and traumatic brain injury' CRIT. CARE vol. 13, no. 6, 2009, page R206, XP021070805

## Description

### Background

Blood flow to the organs may be compromised in a variety of surgical and non-surgical conditions and procedures. Ischemic complications including death may occur.

The satisfactory treatment of, for example, traumatic brain injury (TBI) and/ or spinal cord injury represents a major unmet clinical need. It has been estimated that each year, in the United States alone, approximately 1.5 million people will sustain TBI. At least 15% will be hospitalized and 3% will die. For approximately 80,000 of those that are hospitalized and survive, the injury will herald the onset of long-term disability. A significant number of those that are injured but are not admitted to hospital are also likely to suffer significant health care problems.

One of the difficulties in developing strategies to treat brain or spinal cord injury is the highly heterogeneous nature of both the initial injury and its subsequent pathological development. Severe life-threatening head trauma will inevitably involve mechanisms of developing injury, which differ from those that occur following a mild contusion. Nonetheless, a number of common neuronal and biochemical mechanisms are thought to be involved. It seems to be generally agreed that, while the primary injury will cause immediate mechanical damage to both the vasculature and to neuronal tissue, there follows a complex series of interacting injury cascades driven by, among other things, ischemia, cerebral and spinal chord edema and axotomy. The fact that these processes lead to a developing "secondary" injury has given some hope that interventions can be devised which arrest the development of injury or minimize its impact. Similar mechanisms are described for ischemia of other organs with devastating effects.

Kitaguchi et al., Anesthesiology, vol. 79, no. 4 (1993) describes the induction of anesthesia with thiopental, maintenance with sevoflurane, and the study of the effect of sevoflurane on cerebral blood flow using the Kety-Schmidt method. During the Kety-Schmidt method, argon was used briefly after the surgical procedure and anesthesia as a non-reactive blood flow indicator. This document does not relate to organ protection or co-administration of liquid anesthetic and argon.

WO 2011/098698 relates to a gaseous composition to treat peripheral organ deficiencies. It does not relate to any anesthetic agents or surgery. In this document, an argon atmosphere is maintained around an excised organ before the organ is transplanted to a recipient. The organ donor and the recipient do not receive argon during surgery.

### Summary of the Disclosure

The following summary is provided to introduce the reader to the more detailed discussion to follow. The summary is not intended to limit or define the claims.

With the growing importance of protective strategies, the following disclosure relates to the protection of organs in humans and animals from the side effects of modern liquid anesthetic agents, and/or the devastating effects of glucose and/or oxygen deprivation. Certain side effects of liquid anesthetic agents are discussed in Tang et al., 2013; Kundra et al., 2011; Regueiro-Purrinos et al., 2011; Tetrault et al., 2008; Hu et al., 2014; Liu et al, 2010; and Tong et al., 2013.

Delivering anesthesia is based on delivering at least one of sedation, sleep and pain control. Anesthetics can be gaseous (e.g. xenon and/or nitrous oxide) or liquid (i.e. buprenorphine, propofol, barbiturates, sevoflurane, isoflurane, desflurane). The liquid anesthetic agents can be in the form of a solution, emulsion or any other liquid composition. The liquid anesthetic agents can be delivered orally, directly into the blood stream, or by inhalation. To administer such liquid anesthetic agents by inhalation, they are vaporized. The subset of liquid anesthetic agents that are vaporized to be administered by inhalation are called anesthetic vapors.

The present application relates to the synergistic application of argon, a protective gas which provides no anesthetic effect at atmospheric pressure (referred to herein as the non-anesthetic protective gas), and liquid anesthetic agents, where the organs are protected by the non-anesthetic protective gas, and the liquid anesthetic agents provide the anesthetic component absent in the non-anesthetic protective gas.

The non-anesthetic protective gas may provide some protection against the side effects of the liquid anesthetic agents, and also against the harmful effects of oxygen and glucose deprivation.

The invention provides a non-anesthetic protective gas and a liquid anesthetic agent for use in a method of providing anesthesia and organ-protection to a subject in need thereof, said method comprising co-administering to the subject the non-anesthetic protective gas in an amount effective to provide organ protection, and the liquid anesthetic agent in an amount effective to provide anesthesia, at normobaric conditions, and wherein the non-anesthetic protective gas is argon.

The argon may optionally be administered at a concentration of between 1% and 79%.

In some examples, the liquid anesthetic agent may be administered in liquid form. For example the liquid anesthetic agent may be administered intravenously. In some such examples, the liquid anesthetic agent may be thiopental, propofol, ketamine, hypnomidate, barbiturate, buprenorphine, or dexmedetomidine.

In some examples, the liquid anesthetic agent may be administered in a vapor state. In some such examples, the liquid anesthetic agent may be sevoflurane, isoflurane or desflurane. In some particular examples, the liquid anesthetic agent may be sevoflurane.

In some examples, the liquid anesthetic agent may be administered locally to achieve local or regional anesthesia.

In some examples, the non-anesthetic protective gas and the liquid anesthetic agent may be administered to the subject by an oxygenator in a heart-lung machine, a membrane based device, and/or an extracorporeal membrane oxygenation (ECMO) setup.

In some examples, the non-anesthetic protective gas and the liquid anesthetic agent may be administered to the subject by a ventilator.

In some examples, the ventilator may comprise one or more gas separation membranes that selectively retains, sequesters or exhausts the non-anesthetic protective gas from the air exhaled by the subject, and optionally i) when the non-anesthetic protective gas is retained it is available for further use on the patient and ii) when the non-anesthetic protective gas is sequestered or exhausted, it is subsequently recaptured for future use.

In some examples, administration of the non-anesthetic protective gas in combination with the liquid anesthetic agent may reduce damage to the brain, the spinal cord, the kidney, the liver and/or the heart.

In some examples, administration of the non-anesthetic protective gas in combination with the liquid anesthetic agent may reduce damage resulting from hypoxemia, hypoxia, ischemia, cerebral edema or axotomy.

In some examples, the non-anesthetic protective gas and the liquid anesthetic agent may be administered concurrently or sequentially.

In some examples, the liquid anesthetic agent and non-anesthetic protective gas may be administered to a patient that is undergoing surgery, optionally general surgery or trauma surgery, optionally trauma surgery to treat impact trauma, such as traumatic CNS injury (brain injury or spinal cord injury) or traumatic injury to organs in the torso.

In some examples, the non-anesthetic protective gas and the liquid anesthetic agent may be administered in the absence of xenon.

In some examples, the non-anesthetic protective gas and the liquid anesthetic agent may be administered in the absence of nitrous oxide.

In some examples, the non-anesthetic protective gas and liquid anesthetic agent are administered under normothermic conditions.

These and other objects, advantages, and features of the disclosure will become apparent to those skilled in the art as more fully described below.

### Brief Description of Drawings

The drawings included herewith are for illustrating various examples of articles, methods, and apparatuses of the present specification and are not intended to limit the scope of what is taught in any way. In the drawings:
Figure 1 is a graph showing the experimental design matrix of the Examples section below, including a summary of the number of mice (n) used in each condition.

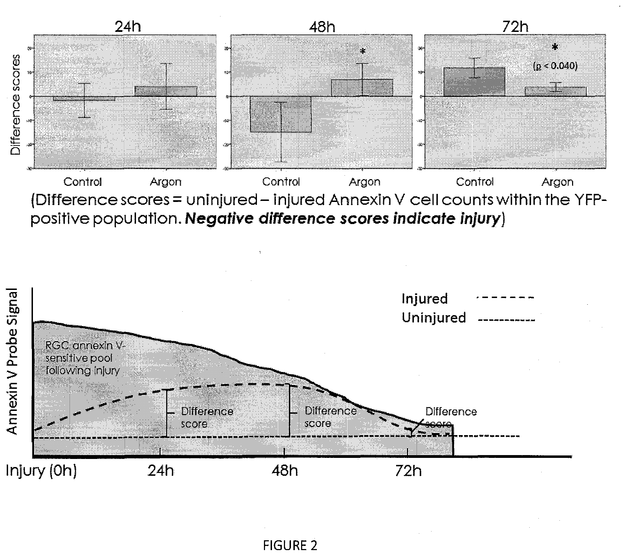

Figure 2 (Top) is a summary of data derived from 24, 48 and 72 hour post-injury analysis. Means and standard errors of difference scores (uninjured - injured numbers of annexin V labeled cells within the YFP population) are plotted. At 24 hours post-injury, early onset of injury is indicated in control mice, as revealed by negative difference scores. Peak neuroprotection by argon is observed at 48 hours, and is retained at 72 hours. (Bottom) Schematic summary describing results for Argon neuroprotection in the context of RGC response to injury. During early induction of apoptosis, the number of RGCs that are responsive to annexin V binding is at its highest, and declines as the apoptotic cascade progresses. Early phase difference scores in injured retinas reflect an increase in annexin V labeling during apoptotic induction. As cells progress through apoptosis, they lose their ability to bind annexin V, thus reducing the pool of annexin V-sensitive cells
Figure 3 (Top) is a descriptive statistics table for the data described in the Examples section below. (Bottom) Independent-samples t-test summary for 72 hour data.

### Detailed Description

The present disclosure relates to normobaric conditions, and in particular the co-administration of a non-anesthetic protective gas which is argon, and liquid anesthetic agents, to provide anesthesia as well as organ-protection. The organ-protection may protect, but is not restricted to, the brain, spinal cord, kidney, liver, lungs, heart and other tissues against damage resulting for example from regional or systemic hypoxemia, inflammation and the subsequent leakage of vessels, anesthetics diminishing autoregulation of blood pressure in the brain, and detrimental effects of by-products (e.g. formaldehyde) created when anesthetics react with chemical absorbers used in anesthetic circuits. In an optional embodiment, the organ-protection is focused on protecting organs of a subject's torso, such as the kidney, liver and heart (torso, or non-neural, organ protection). The non-anesthetic protective gas may provide some protection against the side effects of the liquid anesthetic agents, and also against the harmful effects of oxygen deprivation.

As used herein, the term "liquid anesthetic agent" refers to an anesthetic medicament provided (i.e. distributed and/or administered) in the form of a liquid, such as an aqueous solution, or lipid emulsion. Examples of liquid anesthetic agents include, but are not limited to, medicaments distributed in the form of a liquid, and administered in the form of a vapor. For example, sevoflurane, isoflurane, and desflurane are typically distributed as a liquid, and then vaporized to a vapor state (for example by heating) for administration to a patient via inhalation. The subset of liquid anesthetic agents that are vaporized to a vapor state for administration to a patient may also be referred to as "anesthetic vapors". Further examples of liquid anesthetic agents include, but are not limited to, medicaments distributed in the form of a solid (including bound or encapsulated in a solid), and administered in the form of a liquid, for example by intravenous injection. For example, thiopental is distributed as a powder and is then made up in aqueous solution for administration to a patient. Further examples of liquid anesthetic agents include anesthetic medicaments both distributed and administered in the form of a liquid, such as buprenorphine, barbiturate, propofol, ketamine, hypnomidate, dexmedetomidine.

The term liquid anesthetic agent excludes anesthetic agents distributed and administered in the form of a gas. Examples of anesthetic agents that are distributed and administered in the form of a gas include xenon, and nitrous oxide.

As used herein, the term "non-anesthetic protective gas" refers to gases that provide organ protection, without the ability to provide anesthesia effects under normobaric conditions, such as a sedation, sleep or pain control effects. The non-anesthetic protective gas used in the invention is argon which is known to provide anesthesia at normobaric pressure. Gases that can provide anesthetic effects, such as xenon and nitrous oxide, are excluded from the definition of non-anesthetic protective gases.

As used herein, the term "normobaric" refers to a pressure of about 1 atm (normal air pressure at sea level; approximately 0.1 MPa). It is understood that normobaric conditions will vary according to location and therefore "about" 1 atm will be understood to include naturally occurring variations in atmospheric pressures above or below 1 atm.

As used herein, the term "administration under normobaric conditions" means administration to the patient whilst exposed to a normobaric environment, and includes pressures generated by a typical anesthesia machine, within a cardio pulmonary bypass or by an ECMO setup. Typically this means that exogenous pressure is not applied to the environment by a device, for example a pressure chamber.

As used herein, the term "organ-protection" means protecting an organ or group of organs, such as a brain, spinal cord, kidney etc., against harmful processes such as hypoxemia, impact trauma etc.

As used herein, the term "subject" includes both humans and non-human animals such as mammals, and includes living beings as well as beings that have been declared brain-dead (e.g. prior to organ donation). In some examples, the term "subject" includes a whole body, or part of a body, such as an organ. For example, an organ, after removal from an organ donor and prior to transplant to a recipient, may be considered a "subject". In some examples, the term "subject" may refer to an adult. In some examples, the term "subject" may alternatively or additionally refer to a child. In some examples, the term "subject" may alternatively or additionally refer to an infant. In some examples, the term "subject" may alternatively or additionally refer to a neonate.

The present disclosure provides the use of the non-anesthetic protective gas in combination with a liquid anesthetic agent for co-administration at normobaric pressure, to provide organ-protection. The co-administration is typically pre-administration, concurrent administration, and/or post-administration, in which the non-anesthetic protective gas and liquid anesthetic agent are present in the subject in effective amounts to provide anesthetic effect and organ protection, typically in a synergistic manner.

As noted above, the non-anesthetic protective gas is argon.

It has been found that administering argon under normobaric conditions provides significant neuroprotective effects. Furthermore, administration of argon at high inspiratory concentrations, does not provide anesthetic effects under normobaric conditions. It is believed that this is the first disclosure of i) the synergistic organ-protective effect of the co-administration of a non-anesthetic protective gas which is argon and liquid anesthetics, and ii) the ability of such a combination to overcome the inability of most noble gases to provide anesthesia under normobaric conditions. Xenon at 71% has a capacity to provide anesthesia.

The non-anesthetic protective gas may be administered to a patient as part of a gas mixture that includes oxygen. For example, the concentration for argon may be 79% or less, 70% or less, 60% or less, or 50% or less in oxygen. In some examples, the concentration for argon may be between about 79% and about 1%.

In some examples the liquid anesthetic agent may be administered in the form of a vapor, for use in treatment of a subject in need thereof by administration by inhalation or simulated inhalation. Examples of anesthetic vapors include sevoflurane, isoflurane and desflurane.

As used herein, the term "simulated inhalation" refers to those situations where a patient is or may be unable to achieve sufficient gas exchange using their lungs, and is therefore placed on a heart-lung machine (also known as a cardiopulmonary bypass machine), extracorporeal membrane oxygenation (ECMO) or similar device where a membrane based device allows for the exchange for example of gases and vapors between the blood stream and a gas mixture supplied to the other side of the membrane. In such circumstances, the non-anesthetic protective gas is administered to the oxygenator of the heart-lung machine, which simulates the function of the patient's lungs in allowing oxygen (and the protective gas/ anesthetic vapor) to diffuse into (and carbon dioxide to diffuse out of) blood drawn from the patient. The oxygen-enriched blood is then pumped back to the patient. In such examples, the liquid anesthetic agent may be also be administered via simulated inhalation, or may be administered intravenously.

In some examples, the non-anesthetic protective gas and liquid anesthetic agent are administered under normothermic conditions. For example, the non-anesthetic protective gas and liquid anesthetic agent may be administered at room temperature, without inducing hypothermia.

In some examples, the non-anesthetic protective gas and liquid anesthetic agent may be administered in the absence of any anesthetic gases, such as xenon or nitrous oxide. For example, although trace amounts of xenon and nitrous oxide may be present in the ambient air, no xenon or nitrous oxide are actively administered to the patient.

The methods described herein may be carried out with an apparatus as disclosed in U.S. patent publication no. US 20100031961 A1, entitled "Retention of Noble Gases in The Exhaled Air of Ventilated Patients By Membrane Separation". This publication describes the processing of gas mixtures, in particular, of respiration gases for ventilated patients. The processing according to the disclosure relates, in particular, to the use of selective gas separation membranes for the retention of noble gases in the exhaled air of ventilated patients. The gas separation membrane is a separator, which is integrated in a ventilator or an anesthesia machine. The separation membrane separates the noble gases from the remainder of the residual of the exhaled air by selectively retaining sequestering or exhausting the noble gases as desired. Thus, it is possible to provide a ventilator which enables the application of the non-anesthetic protective gas, i.e. argon, preferably with low loss and as simple as possible.

The methods may also be carried out with an apparatus as disclosed in WO 2007006377 A1, entitled "Device and Method for Preparing Gas Mixtures", also published as US20070017516.

In one embodiment, the non-anesthetic protective gas delivery described above refers to the combined application of the non-anesthetic protective gas in oxygen with an anesthetic vapor. This is typically achieved by inhalation of the non-anesthetic protective gas and the liquid anesthetic as a vapor.

In another embodiment, the non-anesthetic protective gas delivery comprises the administration of a non-anesthetic protective gas, which is stored in pressurized containers until administration to the subject, and the simultaneous or sequential application of a liquid anesthetic agent such as propofol by an intravenous route.

In a further embodiment, the non-anesthetic protective gas/ anesthetic vapor delivery device comprises a heart-lung machine. Operation of such a machine has been briefly described above.

In other examples, the non-anesthetic protective gas may be encapsulated or bound in a compound.

As noted above, in the present disclosure, the term "subject" may in some examples refer to an organ outside of a living body. For example, an organ may be removed from an organ donor for transplant to a recipient. When the organ is in transit (i.e. after removal and prior to transplant), a liquid anesthetic agent and the non-anesthetic protective gas may be administered to the organ. For example, the organ may be stored under an atmosphere that includes an anesthetic vapor and the non-anesthetic protective gas. In such examples, the liquid anesthetic agent may be administered to provide synergistic organ protective effects with the non-anesthetic protective gas.

The embodiments described in this disclosure are representative of the subject matter, which is broadly contemplated by the present invention. The scope of the present invention fully encompasses other embodiments, and the scope of the claims should not be limited by the embodiments set forth in the description and examples, but should be given the broadest interpretation consistent with the description as a whole.

### Examples

### Methods

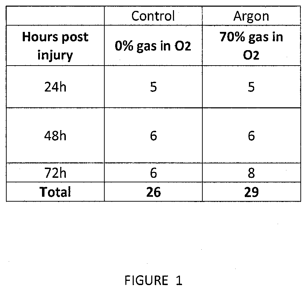

B6.Cg-Tg(Thy1-YFP)16Jrs/J mice: Thy1-YFP mice were exposed to a vapor anesthetic agent (Isofluorane or Sevofluorane), in an anesthetic induction chamber. Animals were temperature probed, sterile eye lubricant was applied to prevent corneal damage, and an incision was made adjacent to the orbital ridge. The left orbit was exposed, and the lacrimal gland gently resected and covered with saline soaked gauze. The superior extraocular muscles were then used to rotate the eye and expose the optic nerve. Using microscissors, the dura was resected, and the optic nerve was transected approximately 0.5mm from the posterior aspect of the eye bulb. Preservation of blood supply was evaluated through the dissecting microscope focused through a dilated pupil and a coverslip. The skin was closed with suture clips, analgesics (buprenorphine in saline) administered, and the mouse was transferred to an Argon (70%)/Oxygen (30%) perfused air chamber on a warming mat. Mice were monitored and housed in ventilated racks for 24 or 72 hours post-injury. Control mice were exposed to normal atmospheric conditions post-injury (See Figure 1). Internal surgical controls were the uninjured retina of the contralateral eye.

Retinal Dissection, dissociation, and staining of cells: Mice were lightly anesthetized with Isofluorane and killed via cervical dislocation. Eyes were rapidly removed and placed in ice cold Hank's Balanced Salt Solution (HBSS). A single puncture to the conjunctiva was made, and corneas removed by microdissection. The ciliary epithelium and lens were then resected, and vitreous humor was visualized using triamcinolone acetonide solution to aid in removal by aspiration and microdissection. Free floating retinas were then transferred to fresh HBSS on ice, then transferred to 1 ml of enzyme solution containing Trypsin (MACS - Miltenyi Biotec). Following a 15 minute incubation at 37 degrees C with periodic agitation, retinas were dissociated with fire polished pipettes and centrifuged. Pellets were washed in Annexin V binding buffer, and centrifuged. Pellets were the re-dissociated in DNAse-containing binding buffer, and stained for Annexin V-PeCy7 probe. Cells were washed, cell strained (40 um), and analyzed using a BD FacsAria III flow cytometer.

Flow cytometry and data analysis: Compensation and gating was established using unstained, YFP-only, Annexin V-only, and double-labeled cell samples. Dead cell assays (not shown) were also performed using propidium iodide and 7-AAD DNA intercalating dyes. Forward and side scatter gating were used to exclude debris, and YFP-positive cells were gated and used as the reference population. Quadrant analysis was used to identify and quantify Annexin V labeling within YFP expressing cells across groups. A within-subjects comparison was performed by comparing injured and uninjured retinas from the same animal. Difference scores (uninjured - injured Annexin V cell counts) were produced in order to normalize Annexin V background labeling in right eyes. All data are represented as means +/- 1.0 standard error of the mean.

Retinal histology: Eyes were enucleated, corneas and lenses removed, and immersed in ice cold 4% paraformaldehyde for 25 minutes. Following 30% sucrose cryoprotection, eyes were introduced to OCT medium, embedded, cut on a cryostat at 14 microns and mounted onto slides. Topro-3 iodide and DRAQ5 nuclear labels were used to evaluate retinal architecture and integrity. YFP and cleaved caspase-3 immunolabeling were used to evaluate early induction of apoptosis in the ganglion cell layer of Thy-1 reporter animals. All imaging was performed on a Zeiss LSM 710 confocal microscope.

### RESULTS

Annexin V-PeCy7 evaluation of early apoptosis in injured retinal ganglion cells reveal a neuroprotective role for argon post-injury treatment.

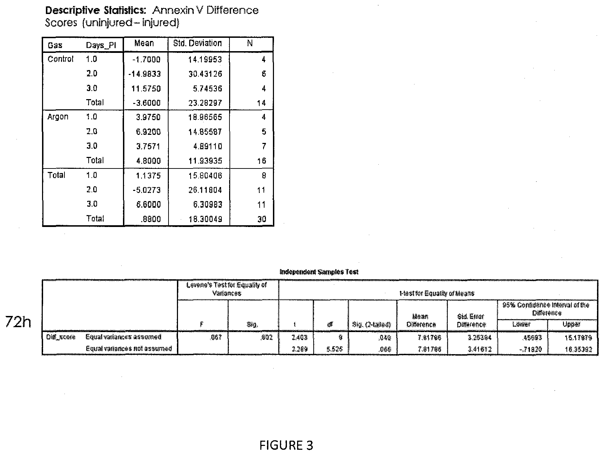

To evaluate the early apoptotic events that follow neural injury, dissociated retinas were probed from Thy-1-YFP reporter mice that underwent unilateral retinal optic nerve axotomy, with a selection of Annexin V dyes. These dyes bind with inner leaflet exposure/externalization of phosphatidylserine that accompanies early apoptosis. Comparing uninjured and injured retinas using flow cytometric counts (500,000 to 1,000,000 events per run) was accomplished by subtracting injured, Annexin V-positive counts from those obtained from uninjured retinas, within the YFP-positive population. At 24h, a slight negative mean difference score in controls indicated that early Annexin V binding events are evident, reflecting an onset of RGC injury (Figures 2 and 3). In contrast to control retinas, injured retinas exposed to argon exhibit a lower level of Annexin V sensitivity, as indicated by a positive difference score, suggesting that the neuroprotective function of argon is present at this time. By 48h, a strong negative difference score in control retinas indicate the peak of Annexin V binding in injured retinas. In contrast to control retinas, injured retinas exposed to Argon maintain a strong resistance to Annexin V binding, suggesting that the peak of neuroprotection effect size resides around 48h post-injury. At 72h post-injury, positive difference scores in control retinas is evident. This observation likely represents a dominance of background labeling that occurs due to sample preparation this time, and a reduction in available RGCs that may be sensitive to Annexin V binding as they have already progressed through early apoptosis.

### Discussion

These data show that argon, when administered in combination with liquid anesthetics, provides a neuroprotective effect within the central nervous system. It is expected that this effect will occur when argon is administered concurrently with liquid anesthetics, prior to liquid anesthetics, and/or subsequent to liquid anesthetics.

It is believed from these data that a larger proportion of RGCs manage to survive over the long term. It is expected that later time points will reveal a sustained population of surviving RGCs following argon exposure.

### References:

Kundra, P., Vinodhadevi, V., & Arimanickam, G. (2011). Sevoflurane-induced arrhythmia in an adult and a child. Journal of Anaesthesiology, Clinical Pharmacology, 27(2), 269-71. doi:10.4103/0970-9185.81844
Regueiro-Purriños, M., Fernandez-Vazquez, F., de Prado, A. P., Altonaga, J. R., Cuellas-Ramon, C., Ajenjo-Silverio, J. M., ... Gonzalo-Orden, J. M. (2011). Ventricular arrhythmias and mortality associated with isoflurane and sevoflurane in a porcine model of myocardial infarction. Journal of the American Association for Laboratory Animal Science : JAALAS, 50(1), 73-8.
Tétrault, S., Chever, O., Sik, A., & Amzica, F. (2008). Opening of the blood-brain barrier during isoflurane anaesthesia. The European Journal of Neuroscience, 28(7), 1330-41. doi:10.1111/j.1460-9568.2008.06443.x
Hu, N., Guo, D., Wang, H., Xie, K., Wang, C., Li, Y., ... Wang, G. (2014). Involvement of the blood-brain barrier opening in cognitive decline in aged rats following orthopedic surgery and high concentration of sevoflurane inhalation. Brain Research, 1551, 13-24. doi:10.1016/j.brainres.2014.01.015
Liu, Y., Ye, Z., Yang, H., Zhou, L., Fan, D., He, S., & Chui, D. (2010). Disturbances of soluble Nethylmaleimide-sensitive factor attachment proteins in hippocampal synaptosomes contribute to cognitive impairment after repetitive formaldehyde inhalation in male rats. Neuroscience, 169(3), 1248-5 doi:10.1016/j.neuroscience.2010.05.061
Tong, Z., Han, C., Luo, W., Wang, X., Li, H., Luo, H., ... He, R. (2013). Accumulated hippocampal formaldehyde induces age-dependent memory decline. Age (Dordrecht, Netherlands), 35(3), 583-96. doi:10.1007/s11357-012-9388-8
Li, X., Zhang, K.-Y., Zhang, P., Chen, L.-X., Wang, L., Xie, M., ... Tang, X.-Q. (2014). Hydrogen sulfide inhibits formaldehyde-induced endoplasmic reticulum stress in PC12 cells by upregulation of SIRT-1. PloS One, 9(2), e89856. doi:10.1371/journal.pone.0089856

## Claims

1. A non-anesthetic protective gas and a liquid anesthetic agent for use in a method of providing anesthesia and organ-protection to a subject in need thereof, said method comprising co-administering to the subject the non-anesthetic protective gas in an amount effective to provide organ protection, and the liquid anesthetic agent in an amount effective to provide anesthesia, at normobaric conditions, and wherein the non-anesthetic protective gas is argon.

2. The non-anesthetic protective gas and liquid anesthetic agent for use according to claim 1 wherein the argon is used at a concentration of between 1% and 79%.

3. The non-anesthetic protective gas and liquid anesthetic agent for use according to claim 1 or claim 2, wherein the liquid anesthetic agent is administered in liquid form, and is optionally administered intravenously.

4. The non-anesthetic protective gas and liquid anesthetic agent for use according to claim 3 wherein the liquid anesthetic agent is thiopental, propofol, ketamine, hypnomidate, barbiturate, buprenorphine, and/or dexmedetomidine.

5. The non-anesthetic protective gas and liquid anesthetic agent for use according to claim 1 or claim 2 wherein the liquid anesthetic agent is administered in a vapor state, and is optionally sevoflurane, isoflurane or desflurane.

6. The non-anesthetic protective gas and liquid anesthetic agent for use according to claim 5 wherein the non-anesthetic protective gas and the liquid anesthetic agent are administered to the subject by a membrane-based device and/or a ventilator.

7. The non-anesthetic protective gas and liquid anesthetic agent for use according to claim 6 wherein the ventilator comprises one or more gas separation membranes that selectively retains, sequesters or exhausts the non-anesthetic protective gas from the air exhaled by the subject, and optionally i) when the non-anesthetic protective gas is retained it is available for further use on the patient and ii) when the non-anesthetic protective gas is sequestered or exhausted, it is subsequently recaptured for future use.

8. The non-anesthetic protective gas and liquid anesthetic agent for use according to any one of claims 1 to 7 wherein administration of the non-anesthetic protective gas in combination with the liquid anesthetic agent reduces damage to the brain, the spinal cord, the kidney, the liver and/or the heart.

9. The non-anesthetic protective gas and liquid anesthetic agent for use according to any one of claims 1 to 8 wherein administration of the non-anesthetic protective gas in combination with the liquid anesthetic agent reduces damage resulting from hypoglycemia, hypoxemia, hypoxia, ischemia, cerebral edema and/or axotomy.

10. The non-anesthetic protective gas and liquid anesthetic agent for use according to any one of claims 1 to 9 wherein the non-anesthetic protective gas and the liquid anesthetic agent are administered concurrently or sequentially.

11. The non-anesthetic protective gas and liquid anesthetic agent for use according to any one of claims 1 to 10 wherein the liquid anesthetic agent and non-anesthetic protective gas are administered to a patient that is intubated and ventilated for stroke, is undergoing surgery, including cardiac surgery, neurosurgery optionally general surgery or trauma surgery, optionally trauma surgery to treat impact trauma, such as traumatic CNS injury (brain injury or spinal cord injury) or traumatic injury to organs in the torso.

12. The non-anesthetic protective gas and liquid anesthetic agent for use according to any one of claims 1 to 11, wherein the non-anesthetic protective gas and the liquid anesthetic agent are administered in the absence of xenon and/or in the absence of nitrous oxide.

13. The non-anesthetic protective gas and liquid anesthetic agent for use according to any one of claims 1 to 12, wherein the non-anesthetic protective gas and liquid anesthetic agent are administered under normothermic conditions.

## Patentansprüche

1. Nicht narkotisches Schutzgas und flüssiges Narkosemittel zur Verwendung in einem Verfahren zum Bereitstellen von Narkose und Organschutz für ein Subjekt, das Bedarf daran hat, wobei das Verfahren gemeinsames Verabreichen an das Subjekt des nicht narkotischen Schutzgases in einer Menge, die effektiv ist, um Organschutz bereitzustellen, und des flüssigen Narkosemittels in einer Menge, die effektiv ist, um Narkose bereitzustellen, unter normobaren Bedingungen umfasst, und wobei das nicht narkotische Schutzgas Argon ist.

2. Nicht narkotisches Schutzgas und flüssiges Narkosemittel zur Verwendung nach Anspruch 1, wobei das Argon bei einer Konzentration zwischen 1% und 79% verwendet wird.

3. Nicht narkotisches Schutzgas und flüssiges Narkosemittel zur Verwendung nach Anspruch 1 oder Anspruch 2, wobei das flüssige Narkosemittel in flüssiger Form verabreicht wird und optional intravenös verabreicht wird.

4. Nicht narkotisches Schutzgas und flüssiges Narkosemittel zur Verwendung nach Anspruch 3, wobei das flüssige Narkosemittel Thiopental, Propofol, Ketamin, Hypnomidat, Barbiturat, Buprenorphin und/oder Dexmedetomidin ist.

5. Nicht narkotisches Schutzgas und flüssiges Narkosemittel zur Verwendung nach Anspruch 1 oder Anspruch 2, wobei das flüssige Narkosemittel in einem Gaszustand verabreicht wird und optional Sevofluran, Isofluran oder Desfluran ist.

6. Nicht narkotisches Schutzgas und flüssiges Narkosemittel zur Verwendung nach Anspruch 5, wobei das nicht narkotische Schutzgas und das flüssige Narkosemittel dem Subjekt durch eine Membran-basierte Vorrichtung und/oder einen Ventilator verabreicht werden.

7. Nicht narkotisches Schutzgas und flüssiges Narkosemittel zur Verwendung nach Anspruch 6, wobei der Ventilator eine oder mehrere Gastrennmembrane umfasst, die selektiv das nicht narkotische Schutzgas von der Luft, die von dem Subjekt ausgeatmet wird, zurückhalten, binden oder ausstoßen, und optional i) wenn das nicht narkotische Schutzgas zurückgehalten wird, es zur weiteren Verwendung am Patienten verfügbar ist und ii) wenn das nicht narkotische Schutzgas gebunden oder ausgestoßen wird, es anschließend zur zukünftigen Verwendung wieder eingefangen wird.

8. Nicht narkotisches Schutzgas und flüssiges Narkosemittel zur Verwendung nach einem der Ansprüche 1 bis 7, wobei Verabreichung des nicht narkotischen Schutzgases in Kombination mit dem flüssigen Narkosemittel Schaden an dem Hirn, dem Rückenmark, der Niere, der Leber und/oder dem Herz verringert.

9. Nicht narkotisches Schutzgas und flüssiges Narkosemittel zur Verwendung nach einem der Ansprüche 1 bis 8, wobei Verabreichung des nicht narkotischen Schutzgases in Kombination mit dem flüssigen Narkosemittel Schaden verringert, der aus Hypoglykämie, Hypoxämie, Hypoxie, Ischämie, Hirnödem und/oder Axotomie resultiert.

10. Nicht narkotisches Schutzgas und flüssiges Narkosemittel zur Verwendung nach einem der Ansprüche 1 bis 9, wobei das nicht narkotische Schutzgas und das flüssige Narkosemittel gleichzeitig oder nacheinander verabreicht werden.

11. Nicht narkotisches Schutzgas und flüssiges Narkosemittel zur Verwendung nach einem der Ansprüche 1 bis 10, wobei das flüssige Narkosemittel und nicht narkotische Schutzgas einem Patienten verabreicht werden, der wegen eines Schlaganfalls intubiert und beatmet wird, sich einem chirurgischen Eingriff unterzieht, einschließlich Herzchirurgie, Neurochirurgie, optional allgemeine Chirurgie, oder Unfallchirurgie, optional Unfallchirurgie zur Behandlung von Schlagtraumata, wie traumatische ZNS-Verletzungen (Hirnverletzung oder Rückenmarksverletzung) oder traumatische Verletzungen von Organen im Rumpf.

12. Nicht narkotisches Schutzgas und flüssiges Narkosemittel zur Verwendung nach einem der Ansprüche 1 bis 11, wobei das nicht narkotische Schutzgas und das flüssige Narkosemittel in der Abwesenheit von Xenon und/oder in der Abwesenheit von Stickstoffoxid verabreicht werden.

13. Nicht narkotisches Schutzgas und flüssiges Narkosemittel zur Verwendung nach einem der Ansprüche 1 bis 12, wobei das nicht narkotische Schutzgas und flüssige Narkosemittel unter normothermen Bedingungen verabreicht werden.

## Revendications

1. Gaz protecteur non anesthésique et agent liquide anesthésique pour une utilisation dans un procédé consistant à fournir une anesthésie et une protection d'organe à un sujet en ayant besoin, ledit procédé comprenant l'étape consistant à co-administrer au sujet le gaz protecteur non anesthésique en une quantité efficace pour fournir une protection d'organe, et l'agent liquide anesthésique en une quantité efficace pour fournir une anesthésie, dans des conditions normobariques, et dans lesquels le gaz protecteur non anesthésique est de l'argon.

2. Gaz protecteur non anesthésique et agent liquide anesthésique pour une utilisation selon la revendication 1, dans lesquels l'argon est utilisé à une concentration entre 1 % et 79 %.

3. Gaz protecteur non anesthésique et agent liquide anesthésique pour une utilisation selon la revendication 1 ou la revendication 2, dans lesquels l'agent liquide anesthésique est administré sous forme liquide, et est facultativement administré par voie intraveineuse.

4. Gaz protecteur non anesthésique et agent liquide anesthésique pour une utilisation selon la revendication 3, dans lesquels l'agent liquide anesthésique est le thiopental, le propofol, la kétamine, l'hypnomidate, le barbiturique, la buprénorphine, et/ou la dexmédétomidine.

5. Gaz protecteur non anesthésique et agent liquide anesthésique pour une utilisation selon la revendication 1 ou la revendication 2, dans lesquels l'agent liquide anesthésique est administré à l'état de vapeur, et est facultativement le sévoflurane, l'isoflurane ou le desflurane.

6. Gaz protecteur non anesthésique et agent liquide anesthésique pour une utilisation selon la revendication 5, dans lesquels le gaz protecteur non anesthésique et l'agent liquide anesthésique sont administrés au sujet par un dispositif à membrane et/ou un respirateur.

7. Gaz protecteur non anesthésique et agent liquide anesthésique pour une utilisation selon la revendication 6, dans lesquels le respirateur comprend une ou plusieurs membranes de séparation de gaz qui retiennent, emprisonnent ou évacuent sélectivement le gaz protecteur non anesthésique de l'air expiré par le sujet, et facultativement i) lorsque le gaz protecteur non anesthésique est retenu, il est disponible pour une utilisation ultérieure sur le patient et ii) lorsque le gaz protecteur non anesthésique est emprisonné ou évacué, il est recapturé ultérieurement pour une utilisation future.

8. Gaz protecteur non anesthésique et agent liquide anesthésique pour une utilisation selon l'une quelconque des revendications 1 à 7, dans lesquels l'administration du gaz protecteur non anesthésique en combinaison avec l'agent liquide anesthésique réduit les dommages au cerveau, à la moelle épinière, au rein, au foie et/ou au cœur.

9. Gaz protecteur non anesthésique et agent liquide anesthésique pour une utilisation selon l'une quelconque des revendications 1 à 8, dans lesquels l'administration du gaz protecteur non anesthésique en combinaison avec l'agent liquide anesthésique réduit les dommages résultant de l'hypoglycémie, l'hypoxémie, l'hypoxie, l'ischémie, l'œdème cérébral et/ou l'axotomie.

10. Gaz protecteur non anesthésique et agent liquide anesthésique pour une utilisation selon l'une quelconque des revendications 1 à 9, dans lesquels le gaz protecteur non anesthésique et l'agent liquide anesthésique sont administrés simultanément ou séquentiellement.

11. Gaz protecteur non anesthésique et agent liquide anesthésique pour une utilisation selon l'une quelconque des revendications 1 à 10, dans lesquels l'agent liquide anesthésique et le gaz protecteur non anesthésique sont administrés à un patient qui est intubé et ventilé pour une congestion cérébrale, subit une chirurgie, incluant une chirurgie cardiaque, une neurochirurgie, facultativement une chirurgie générale ou une chirurgie traumatologique, facultativement une chirurgie traumatologique pour traiter un traumatisme par impact, tel qu'un dommage au système nerveux central (traumatisme crânien ou lésion de la moelle épinière) ou un traumatisme à des organes dans le torse.

12. Gaz protecteur non anesthésique et agent liquide anesthésique pour une utilisation selon l'une quelconque des revendications 1 à 11, dans lesquels le gaz protecteur non anesthésique et l'agent liquide anesthésique sont administrés en l'absence de xénon et/ou en l'absence d'oxyde nitreux.

13. Gaz protecteur non anesthésique et agent liquide anesthésique pour une utilisation selon l'une quelconque des revendications 1 à 12, dans lesquels le gaz protecteur non anesthésique et l'agent liquide anesthésique sont administrés dans des conditions normothermiques.
